# EUROPEAN PATENT APPLICATION

(11) **EP 4 785 992 A1**
(43) Date of publication of application: **05.08.2026**
(21) Application number: 24870050.2
(22) Date of filing: 25.07.2024
(51) Int. Cl.: A61N 1/372, A61N 1/375

(54) **MEDICAL DEVICE AND MEDICAL DEVICE CONTROL SYSTEM**

(30) Priority: 26.09.2023 CN 202311251689; 26.09.2023 CN 202322633658 U
(71) Applicant: MicroPort Soaring CRM (Shanghai) Co., Ltd., Shanghai 201203 (CN)
(72) Inventor: ZHU, Xiaoming, Shanghai 201203 (CN); LIAO, Wangcai, Shanghai 201203 (CN); WU, Nan, Shanghai 201203 (CN); CHENG, Zhijun, Shanghai 201203 (CN); YU, Peng, Shanghai 201203 (CN)
(74) Representative: Lavoix
(86) International application number: PCT/CN2024/107437
(87) International publication number: WO 2025/066496

(57) **Abstract**

The present invention provides a medical device and a medical device control system. The medical device includes a main enclosure, an electrical assembly, a connection assembly and a patch assembly. Both the electrical assembly and the connection assembly are arranged in the main enclosure, and a first through hole for passage of an electrode lead therethrough is formed in the main enclosure. The connection assembly is configured to be electrically connected to the electrical assembly and the electrode lead, and the patch assembly is configured to fix at least one of the main enclosure and the electrode lead at a target site. The medical device is lighter in weight, compacter in size, more easily wearable by a wearer and associated with a lower risk of infection, and is therefore more favorable to the wearer's recovery.

## Description

### TECHNICAL FIELD

The present invention relates to the field of medical devices and, in particular, to a medical device and a medical device control system.

### BACKGROUND

Nowadays, temporary (provisional) implantable medical devices have found extensive use. For example, postoperative wearing of a temporary pacemaker can obviate the wearer from both bradycardia-related adverse events and premature, unnecessary implantation of a permanent pacemaker. These devices primarily address the following three categories of needs:
1) Preventive need: any potential need for protection by pacing during a perioperative period, for example, generally after surgery including surgical valve replacement, transcatheter valve intervention, radiofrequency arrhythmia ablation, infected lead extraction and surgical resection or chemical ablation for hypertrophic cardiomyopathy.
2) Therapeutic need: any need for temporary pacing for therapeutic purposes, such as indications for pacing after any surgery, for which recovery is unpredictable, such as acute myocardial infraction and percutaneous coronary intervention (PCI).
3) Provisional need: therapeutic protection over an intermediate interval from the extraction of a previously implanted pacemaker due to any reason, such as an infection, to the implantation of a new pacing system for replacement.

However, conventional temporary pacemakers are bulky and, during use, would restrict a wearer's physical activities, whether hanging on an intravenous (IV) stand or placed by the wearer's bedside, which is detrimental to the wearer's recovery.

In order to overcome this problem, a provisional pacemaker has been proposed in the related art, which includes an electrode lead adapted for implantation in a wearer's body and an impulse generator connected to the electrode lead. The impulse generator is provided with attachment lugs, with which the impulse generator can be sewn onto or fixed beneath the wearer's skin. Although this design less hinders a wearer's physical activities, it requires sewing the impulse generator onto the wearer's skin, increasing the risk of trauma and infection. Moreover, it can be used with only a few types of electrode leads, failing to provide a physician with a sufficient number of options for addressing different preventive, therapeutic or provisional needs of individual wearers.

A fixation device known in the related art can be used to fix a temporary pacing lead on a wearer's body surface essentially based on the use of a strap in combination with hook-and-loop fasteners. Despite less restricted physical activities of a wearer, this design is specific to the fixation of a temporary pacing lead and fails to solve the problem of fixing a conventional temporary pacemaker, which is bulky, large in size and not easy to wear.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

The present invention seeks to overcome one or more of the above-described problems associated with conventional temporary pacemakers, including high bulkiness, a lack of ease of wearing and a high risk of infection, by presenting a medical device, which is lightweight, compact, more easily wearable by a wearer and associated with a reduced risk of infection, and a medical device control system.

To this end, the present invention provides a medical device including a main enclosure, an electrical assembly, a connection assembly and a patch assembly. Both the electrical assembly and the connection assembly are arranged in the main enclosure, and a first through hole adapted for passage of an electrode lead therethrough is provided in the main enclosure.

The connection assembly is configured to be electrically connected to the electrical assembly and the electrode lead.

The patch assembly is configured to fix at least one of the main enclosure and the electrode lead to a target site.

Optionally, the electrical assembly may include an electrical protection enclosure, a power supply and a control-system circuit module electrically connected to the power supply, wherein the power supply and the control-system circuit module are disposed in a closed accommodation space delimited by the electrical protection enclosure and the main enclosure, and
the power supply is configured to provide electrical power to the control-system circuit module.

Optionally, the electrical assembly may further include a connection cable, which electrically connects the control-system circuit module to the connection assembly, wherein the electrical protection enclosure defines a second through hole therein adapted for passage of the connection cable therethrough.

Optionally, the electrical protection enclosure may further include a sealing member for sealing a gap between the second through hole and the connection cable.

Optionally, the connection assembly may include at least one mounting recess and at least one electrical connector, wherein the at least one mounting recess is configured to receive a connector of the electrode lead therein, and wherein the connector of the electrode lead is electrically connected to the electrical assembly by the at least one electrical connector.

Optionally, the main enclosure may include an upper shell, a lower shell and a locking mechanism, wherein the upper shell is articulated to the lower shell, making the main enclosure of a flip-top type, or the upper shell is slidably coupled to the lower shell, making the main enclosure of a slide-top type; and
wherein the upper and lower shells, when closed together, are locked to each other by the locking mechanism.

Optionally, the upper shell of the main enclosure may be provided with at least one recessed accommodation portion corresponding to the respective electrical connector one-to-one, wherein the at least one electrical connector is partially received in respective recessed accommodation portion, and wherein the at least one mounting recess is provided in the lower shell of the main enclosure.

Optionally, each of the at least one recessed accommodation portion may be a blind hole extending perpendicular to a plane in which the upper shell extends,
wherein each electrical connector includes:
a contact pin, which defines a first spring engagement feature extending around its circumference;
a connecting sheet attached to the upper shell of the main enclosure, which extends in a direction forming a non-zero angle with a direction of extension of the contact pin, and through which the contact pin is passed; and
a first spring, which is disposed over the contact pin, with its opposite ends being respectively in abutment with the first spring engagement feature and the blind hole,
wherein when the electrical connector is electrically connected to the connector of the electrode lead, the contact pin is located at a first position, and the first spring is in a first compressed configuration, in which it has a first length, with a gap being left between the first spring engagement feature and the connecting sheet, and with the contact pin being in abutment with the connector at an end thereof away from the blind hole;
when the electrical connector is disconnected from the connector of the electrode lead, the contact pin is located at a second position, and the first spring is in a second compressed configuration, in which it has a second length, with the first spring engagement feature being in abutment with the connecting sheet; and
the second length is greater than the first length.

Optionally, the recessed accommodation portion may be a through-hole structure extending perpendicular to a plane in which the upper shell extends,
wherein each electrical connector includes:
a set screw, which defines a second spring engagement feature extending around its circumference;
a conductive connecting block attached to the upper shell of the main enclosure, which defines a third through hole in alignment with the through-hole structure and in threaded engagement with the set screw, and of which the second spring engagement feature is located on a side away from the lower shell of the main enclosure; and
a second spring, which is disposed over the set screw, with its opposite ends being respectively in abutment with the second spring engagement feature and the conductive connecting block,
wherein the set screw, when rotated, changes its position relative to the conductive connecting block, thereby switching the electrical connector between a connected state, in which it is electrically connected to the connector of the electrode lead, and a disconnected state.

Optionally, the medical device may further include a cable auxiliary fixing member disposed inside the main enclosure.

Optionally, the medical device may further include:
a sealing ring disposed at a location where the upper and lower shells of the main enclosure come into abutment with each other; and/or
a sealing pad for sealing a gap between the electrode lead and the first through hole, the sealing pad including a silicone pad having a cross-shaped cut therein, through which the connector of the electrode lead is passed and connected to the connection assembly.

Optionally, the patch assembly may include at least one first patch, which is adapted to fix the main enclosure at the target site, and the first patch is detachably attached to the main enclosure.

Optionally, the patch assembly may include a single tearable first patch having a fourth through hole therein, which is adapted for passage of the electrode lead therethrough.

Alternatively, the patch assembly may include at least two first patches, which are individually detachably attached to the main enclosure and thereby fix the main enclosure at the target site, wherein a gap allowing passage of the electrode lead therethrough is left between the at least two first patches.

Optionally, the patch assembly may include a main patch and an auxiliary adhesive patch capable of connecting to the target site, wherein the main enclosure and a portion of the electrode lead are disposed between the main patch and the auxiliary adhesive patch.

Optionally, the main enclosure may be separate from the patch assembly and wearable, wherein the patch assembly includes a second patch for fixing the electrode lead at the target site.

Optionally, the main enclosure may include at least two separate sub-enclosures, the electrical assembly, the connection assembly and a portion of the electrode lead are accommodated in the at least two separate sub-enclosures, wherein the connection assembly and the portion of the electrode lead are housed in a single one of the sub-enclosures.

Optionally, the medical device may further include a tuning assembly disposed on an outer wall surface of the main enclosure, the tuning assembly including at least one adjustment knob electrically connected to the electrical assembly.

To the above end, the present invention also provides a medical device control system including a medical programming device and the medical device as defined above, which is communicatively connected to the medical programming device.

The present invention offers the following benefits over the prior art:
It provides a medical device including a main enclosure, an electrical assembly, a connection assembly and a patch assembly. The electrical assembly and the connection assembly are arranged in the main enclosure, and the main enclosure defines a first through hole adapted for passage of an electrode lead therethrough. The connection assembly is configured to be electrically connected to the electrical assembly and the electrode lead, and the patch assembly is configured to fix at least one of the main enclosure and the electrode lead at a target site (for example, when the patch assembly is used to fix the main enclosure, the target site may be on the body surface of a wearer of the medical device). Thus, through integrating the main enclosure, the electrical assembly, the connection assembly and the patch assembly, the medical device of the present invention combines the advantages of both implantable medical devices (e.g., implantable pacemakers) and temporary medical devices (e.g., conventional temporary pacemakers) in the related art. With this design, the inventive medical device is lighter in weight, compacter in size, more easily wearable by a wearer and associated with a lower risk of infection, and is therefore more favorable to the wearer's recovery. Moreover, through housing the electrical assembly and the connection assembly in the main enclosure, not only protection can be provided for the electrical assembly and the connection assembly, but a main body of the medical device (consisting of the main enclosure, the electrical assembly and the connection assembly) can be worn on the wearer through the patch assembly. Additionally, since the main body of the medical device is lightweight and compact, it can be worn, for example, via a back or other strap, or even placed within a pocket. Thus, the patch assembly can be used to fix an electrode lead and take care of and provide protection for an incision made at a puncture site (i.e., the target site), reducing the risk of infection at the puncture site. Further, in the medical device of the invention, the connection assembly can be suitably used with a variety of electrode leads (e.g., it is adapted to mate with connectors of standard IS-1 electrode leads, connectors of floating electrodes for temporary pacing, connecting pins of temporary pacing electrodes, etc.) Because of such applicability to more electrode leads, a physician is allowed to more easily make an appropriate recovery plan (e.g., preventive, therapeutic or temporary (provisional), using the proposed inventive medical device) that matches the actual need of an individual wearer.

The present invention also provides a medical device control system, which belongs to the same inventive concept as the medical device of the invention and thereby has at least all the advantages of the inventive medical device. For brevity, these advantages are not repeated here, and reference is made to the above description in connection with the medical device for more details.

### BRIEF DESCRIPTION OF THE DRAWINGS

Fig. 1 shows a schematic overall view of a medical device embodying the present invention.
Fig. 2 shows a schematic illustration of a medical device according to a first embodiment of the present invention.
Fig. 3 schematically illustrates an example of the medical device according to the first embodiment of the present invention being connected to an implantable IS-1 ventricular electrode lead.
Fig. 4 schematically illustrates another example of the medical device according to the first embodiment of the present invention being connected to implantable IS-1 ventricular and atrial electrode leads.
Fig. 5 schematically illustrates yet another example of the medical device according to the first embodiment of the present invention being connected to a conventional temporary pacing electrode lead.
Fig. 6 shows an example layout of the medical device according to the first embodiment of the present invention and an electrode lead connected thereto inside a main enclosure.
Fig. 7 shows another example layout of the medical device according to the first embodiment of the present invention and the electrode lead connected thereto inside the main enclosure.
Fig. 8 shows a cross-sectional view of an example of a connection assembly in a medical device according to a second embodiment of the present invention, taken along a first direction.
Fig. 9 shows a perspective view of an example of the connection assembly in the medical device according to the second embodiment of the present invention partially cut away along the first direction.
Fig. 10 shows a perspective partial view of an example of the connection assembly in the medical device according to the second embodiment of the present invention partially cut away along a second direction (perpendicular to the first direction).
Fig. 11 shows a cross-sectional view of a medical device according to a third embodiment of the present invention, taken along a third direction.
Fig. 12 shows a perspective view of the medical device according to the third embodiment of the present invention partially cut away along the third direction.
Fig. 13 shows a perspective partial view of the medical device according to the third embodiment of the present invention partially cut away along a fourth direction (perpendicular to the third direction).
Fig. 14 shows an elevational view of a medical device according to a fourth embodiment of the present invention.
Fig. 15 schematically illustrates an example of the medical device according to the fourth embodiment of the present invention being connected to an implantable IS-1 ventricular electrode lead.
Fig. 16 schematically illustrates another example of the medical device according to the fourth embodiment of the present invention being connected to implantable IS-1 ventricular and atrial electrode leads.
Fig. 17 schematically illustrates yet another example of the medical device according to the fourth embodiment of the present invention being connected to a conventional temporary pacing electrode lead.
Fig. 18 schematically illustrates opened upper and lower shells in a medical device according to a fifth embodiment of the present invention.
Fig. 19 schematically illustrates a main enclosure in a closed configuration and a protective feature for preventing accidental touching in an opened configuration in the medical device of Fig. 18.
Fig. 20 schematically illustrates the main enclosure in the closed configuration and the protective feature for preventing accidental touching also in a closed configuration in the medical device of Fig. 18.
Fig. 21 shows a schematic overall view of a medical device according to a sixth embodiment of the present invention, in which a main enclosure is in an opened configuration.
Fig. 22 is a schematic illustration of the main enclosure in the medical device according to the sixth embodiment of the present invention, which is in a closed configuration.
Fig. 23 is a schematic illustration of the main enclosure in the medical device of Fig. 22, which is in the opened configuration.

### List of Reference Numerals

100, main enclosure; 110, upper shell; 120, lower shell; 130, locking mechanism;
200 electrical assembly; 210 electrical protection enclosure; 211 stop tab; 220 power supply; 230 control-system circuit module; 240 tuning assembly; 241 adjustment knob; 242 protective feature for preventing accidental touching;
300 connection assembly; 310 mounting recess; 320 electrical connector; 321a blind hole; 321b contact pin; 321b1 first spring engagement feature; 321c first spring; 321d connecting sheet; 322a through-hole structure; 322b set screw; 322b1second spring engagement feature; 322b2 external thread; 322c second spring; 322d conductive connecting block;
410 first patch; 421 main patch; 422 auxiliary adhesive patch;
510 sealing ring; 520 sealing pad;
600 electrode lead; 610 IS-1 ventricular electrode lead; 620 IS-1 atrial electrode lead; 630 temporary electrode lead;
P-TPG patch-type temporary pacemaker;
700 heart.

### DETAILED DESCRIPTION

Objects, advantages and features of the present invention will become more apparent upon reading the following more detailed description of medical devices and medical device control systems proposed herein in conjunction with the accompanying drawings. Note that the figures are rather simplified and not necessarily to scale, with the only intention to help explain embodiments of the invention disclosed herein in a more convenient and clearer way. It will be understood that the figures may not necessarily illustrate the structures described herein to scale and that illustrative features depicted in the figures to explain certain principles of the present invention may be slightly simplified. The specific design features of the invention as disclosed herein, including, for example, specific dimensions, orientations, locations and shapes, will be determined in part by the particular intended application and use environment. Additionally, in the embodiments described below, like reference numerals may be sometimes used to refer to the same or functionally similar elements throughout different figures, while description thereof may not be repeated. In this specification, similar reference numerals and letters refer to similar items in the figures, and thus once an item is defined in one figure, it may not be discussed for following figures. Where appropriate, the terms so used are interchangeable.

It is to be noted that relational terms such as "first," "second," and the like may be used herein solely to distinguish one entity or action from another entity or action, without necessarily requiring or implying any actual such relationship or order between such entities or actions, or denoting or implying relative importance of them, or implicitly indicating the number of them. Moreover, the terms "comprise", "include" and any variations thereof are intended to cover a non-exclusive inclusion, such that a process, method, article, or apparatus that includes a list of elements is not necessarily limited to those elements, but may include other elements not expressly listed or inherent to such process, method, article, or apparatus. An element preceded by "comprises... a" does not, without more constraints, preclude the existence of additional identical elements in the process, method, article, or apparatus that includes the element. As used herein, the singular forms "a", "an" and "the" include plural referents, and the term "or" is generally employed in the sense of "and/or", "a number of" of "at least one", and "at least two" of "two or more". In addition, the terms "first," "second," and "third" are intended only for illustration and are not to be construed as indicating or implying relative importance, or as implicitly indicating the number of the referenced items.

It is to be noted that, as used herein, the terms "central", "longitudinal", "transverse", "length", "width", "thickness", "upper", "lower", "front", "rear", "left", "right", "vertical", "horizontal", "top", "bottom", "inner", "outer", "axial", "radial", "circumferential" and so on may be used herein to describe orientations or positional relationships as viewed in the annexed figures. They are for convenience and ease of description of the present invention only and do not indicate or imply that the described apparatus or element must assume, or be constructed or operated in, a particular orientation. Therefore, they are not to be construed as limiting the present invention.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements, unless otherwise clearly defined. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature .When a first feature is referred to as being "under", "below" or "at bottom of" a second feature, it may be right or obliquely under, below or at bottom of the second feature, or simply located at a lower level than the second feature .

It should be particularly noted that medical devices and medical device control systems are proposed herein, which serve as provisional (temporary) replacements for various implantable medical devices, and the present invention is not limited to any particular type of implantable medical device, or to any particular implantation site. For example, such an implantable medical device may be any of a cardiac defibrillator, a cardiac pacemaker, a leadless pacemaker and a deep brain stimulator. For ease of understanding, the proposed implantable medical devices are described herein in the context of implantable cardiac pacemakers, as an example.

### EXAMPLE 1

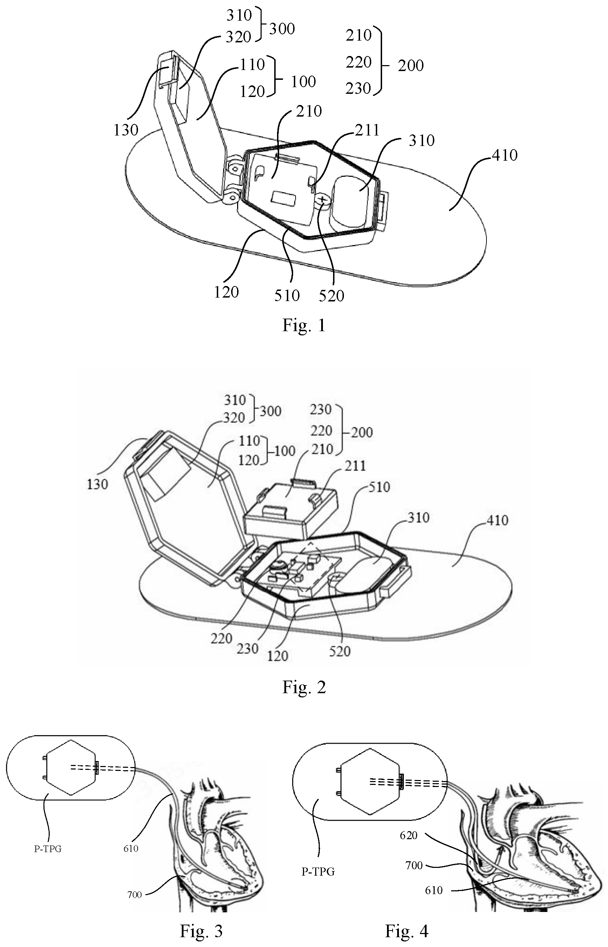

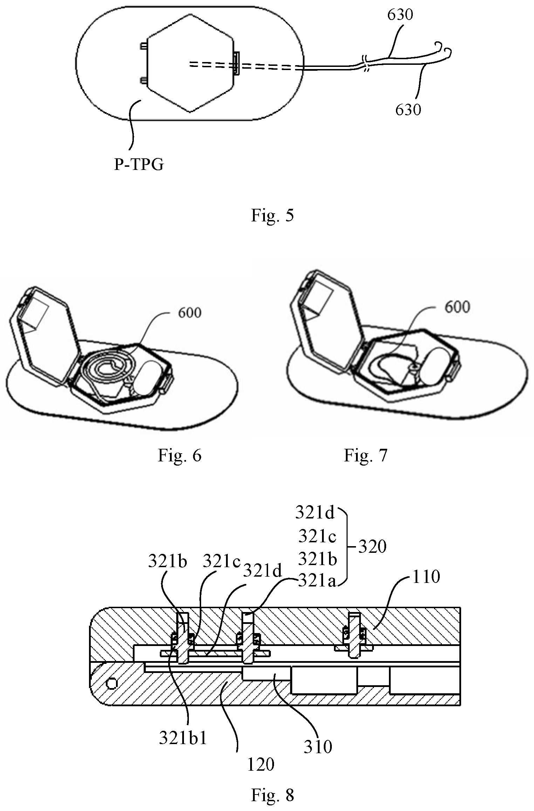

A first embodiment provides a medical device, which is a patch-type temporary pacemaker. Particular reference is now made to Figs. 1 and 2. Fig. 1 shows a schematic overall view of the medical device of this embodiment. Fig. 2 shows a schematic illustration of the medical device of this embodiment. As can be seen from Figs. 1 and 2, the medical device of this embodiment includes a main enclosure 100, an electrical assembly 200, a connection assembly 300 and a patch assembly (including a first patch 410 in Fig. 1). The electrical assembly 200 and the connection assembly 300 are both arranged in the main enclosure 100, and a first through hole (not labeled) for passage of an electrode lead 600 (not shown in Figs. 1 and 2, but can be best seen in Fig. 6 or 7) therethrough is formed in the main enclosure 100. The connection assembly 300 is configured to be electrically connected to the electrical assembly 200 and an electrode lead 600. The patch assembly is configured to fix the main enclosure 100 at a target site.

In other specific implementations, the patch assembly can be used to fix either only an electrode lead 600, or both the main enclosure 100 and an electrode lead 600, at a target site. Further description of these implementations is deemed unnecessary and omitted herein.

Notably, as would be appreciated by those skilled in the art, the "target site" as mentioned herein may include, but is not limited to, a location on a wearer's body surface, where the medical device is placed in the case of the patch assembly being used to fix the fixation main enclosure 100, or an incision on the wearer in the case of the patch assembly being used to fix an electrode lead 600 and to take care of and provide protection for the incision. In the latter case, the patch assembly may include a second patch, with which an electrode lead 600 is fixed, and the main enclosure 100 may be a wearable enclosure and positioned on a back or other strap worn on the wearer, or in a pocket.

Thus, through integrating the main enclosure 100, the electrical assembly 200, the connection assembly 300 and the patch assembly, the medical device of this embodiment combines the advantages of both implantable medical devices (e.g., implantable pacemakers) and temporary medical devices (e.g., conventional temporary pacemakers) in the related art. With this design, the inventive medical device is lighter in weight, compacter in size, more easily wearable by a wearer and associated with a lower risk of infection, and is therefore more favorable to the wearer's recovery. Moreover, through housing the electrical assembly 200 and the connection assembly 300 in the main enclosure 100, not only protection can be provided for the electrical assembly 200 and the connection assembly 300, but a main body of the medical device (consisting of the main enclosure 100, the electrical assembly 200 and the connection assembly 300) can be worn on the wearer through the patch assembly. Additionally, since the main body of the medical device is lightweight and compact, it can be worn, for example, via a back or other strap, or even placed within a pocket. Thus, the patch assembly can be used to fix an electrode lead and take care of and provide protection for an incision made at a puncture site (i.e., the target site), reducing the risk of infection at the puncture site. Further, in the medical device of this embodiment, the connection assembly 300 can be suitably used with a variety of electrode leads 600 (e.g., it is adapted to mate with connectors of standard IS-1 electrode leads, connectors of floating electrodes for temporary pacing, connecting pins of temporary pacing electrodes, etc.) Because of such applicability to more electrode leads 600, a physician is allowed to more easily make an appropriate recovery plan (e.g., preventive, therapeutic or temporary (provisional), using the proposed inventive medical device) that matches the actual need of an individual wearer.

More particular reference is now made to Fig. 2. As can be seen from Fig. 2, in the medical device of this embodiment, the main enclosure 100 includes upper shell 110, a lower shell 120 and a locking mechanism 130. The upper shell 110 is articulated to the lower shell 120, making the main enclosure 100 of a flip-top type. When closed together, the upper shell 110 and the lower shell 120 can be locked to each other using the locking mechanism 130. In this embodiment, the locking mechanism 130 is a snap-fit. Articulating the upper shell 110 to the lower shell 120 in the main enclosure 100 not only imparts high robustness and good durability to the main enclosure 100, but also facilitates the assembly of the medical device and maintenance and debugging of the functional components housed therein (e.g., the electrical assembly 200, the connection assembly 300, etc.) Further, with the locking mechanism 130, the main enclosure 100 can be firmly retained in the closed configuration.

With continued reference to Fig. 1 or 2, in some exemplary implementations, the electrical assembly 200 is arranged on the lower shell 120 of the main enclosure 100. In the medical device of this embodiment, arranging the electrical assembly 200 on the lower shell 120 can not only ensure stable retention of the electrical assembly 200, but can also facilitate closing and opening of the main enclosure 100 by flipping the upper shell 110. Optionally, the first through hole for passage of an electrode lead 600 therethrough may be provided in the lower shell 120, additionally facilitating the securing of the electrode lead 600.

With continued reference to Fig. 2, in some exemplary implementations, the electrical assembly 200 includes an electrical protection enclosure 210, a power supply 220 and a control-system circuit module 230 electrically connected to the power supply 220. The power supply 220 and control-system circuit module 230 are received in a closed accommodation space delimited by the electrical protection enclosure 210 and the main enclosure 100. The power supply 220 serves to provide electrical power to the control-system circuit module 230. Enhanced protection can be provided for the power supply 220 and the control-system circuit module 230 by arranged them within the accommodation space enclosed by the electrical protection enclosure 210 and the main enclosure 100, avoiding them from direct contact with an electrode lead 600. Otherwise, during physical activities of the wearer, the power supply 220 and the control-system circuit module 230 may displace relative to and rub against an electrode lead 600, possibly causing damage to the electrode lead 600, or to the power supply 220 and the control-system circuit module 230. Therefore, the enhanced protection can ultimately extend the service life of the medical device of this embodiment.

Further, as would be appreciated by those skilled in the art, the power supply 220 is included essentially to provide electrical power to the control-system circuit module 230. The present invention is not limited to any particular type of power supply 220. In some example implementations, the power supply 220 may be implemented as a button battery, such as a CR2032 or other lithium battery commonly used in consumer electronics, and mounted in the electrical protection enclosure 210 using a conventional battery holder. Further, as would be appreciated by those skilled in the art, the present invention is not limited to any particular composition of the control-system circuit module 230. The control-system circuit module 230 (as schematically indicated the dashed box in the figure) may include one or more submodules.

In particular, the control-system circuit module 230 may include a medical circuit module (not labeled) configured to monitor the current physiological state of the wearer's organ or tissue, and/or enhance or replace the physiological function of the wearer's impaired organ or tissue. That is, the control-system circuit module 230 functions substantially in the same way as a control-system circuit module 230 of a counterpart implantable medical device (e.g., an implantable cardiac pacemaker) of the medical device. For example, the medical circuit module may have a rate response function, which allows the medical device to increase its pacing rate in response to a need of the patient for a higher heart rate due to accelerated metabolism during physical activities.

For example, in one exemplary implementation, the medical device is provided as a temporary replacement for an implantable cardiac pacemaker (i.e., it is implemented as a patch-type temporary pacemaker, or "P-TPG" for short). In this case, the medical circuit module may further include a pacing control submodule (not labeled) configured to provide pacing at 60 beats per minute (BPM), corresponding to the basic heart rate, or at 180 BPM, as necessary for postoperative recovery after transcatheter aortic valve replacement (TAVR) or transcatheter aortic valve implantation (TAVI).

Optionally, in some exemplary implementations, the control-system circuit module 230 may further include a wireless communication module (not labeled) configured to be communicatively connected to the medical circuit module and a medical programming device. Thus, the inventive medical device (e.g., a P-TPG) is able to communicate with another device (e.g., a telemetry unit, programmer, electrocardiogram monitor, etc.), allowing a medical care provider (e.g., a nurse, attending physician, etc.) to monitor operation of the medical device (e.g., a P-TPG) in real time and adjust its parameters, as needed.

The present invention is not limited to any particular type of wireless communication module for use in the control-system circuit module 230 of the medical device. Possible examples of the wireless communication module may include, but are not limited to, Bluetooth, radio frequency (RF) and WiFi communication modules. Further description thereof is deemed unnecessary and omitted herein.

When the medical device of this embodiment is implemented as a temporary pacemaker (e.g., a P-TPG), suitable electrode leads 600 may include, but are not limited to, an IS-1 ventricular electrode lead 610, an IS-1 atrial electrode lead 620 and a temporary electrode lead 630. Particular reference is now made to Figs. 3 to 5. Fig. 3 schematically illustrates an example of the medical device of this embodiment being connected to an implantable IS-1 ventricular electrode lead. As can be seen from Fig. 3, in this example, when implemented as a patch-type temporary pacemaker (P-TPG), the present inventive medical device may be connected to an IS-1 ventricular electrode lead 610 and used for temporary single-chamber (ventricular) pacing of the wearer's heart 700. Fig. 4 schematically illustrates another example of the medical device of this embodiment being connected to implantable IS-1 ventricular and atrial electrode leads. As can be seen from Fig. 4, in this example, when implemented as a patch-type temporary pacemaker (P-TPG), the inventive medical device may be connected to an IS-1 ventricular electrode lead 610 and an IS-1 atrial electrode lead 620 and used for temporary dual-chamber (atrial and ventricular) pacing of the wearer's heart 700. Fig. 5 schematically illustrates yet another example of the medical device of this embodiment being connected to a conventional temporary pacing electrode lead. As can be seen from Fig. 5, in this example, when implemented as a patch-type temporary pacemaker (P-TPG), the inventive medical device may be connected to a temporary electrode lead 630 and used for temporary pacing of the wearer's heart 700.

Optionally, in some exemplary implementations, the electrical assembly 200 may further include a connection cable (not labeled), which electrically connects the control-system circuit module 230 to the connection assembly 300. In the electrical protection enclosure 210, a second through hole (not labeled) for passage of the connection cable therethrough is formed. Thus, in addition to providing protection for the electrical assembly 200, the electrical protection enclosure 210 allows the connection cable for the control-system circuit module 230 to be passed through the second through hole and connected to the connection assembly 300.

Optionally, in some exemplary implementations, the electrical protection enclosure 210 may further include a sealing member (not labeled) for sealing a gap between the second through hole and the connection cable. Sealing the gap between the second through hole and the connection cable by the sealing member ensures that the electrical protection enclosure 210 sealingly encloses the electrical assembly 200, contributing to an extended service life of the medical device.

It should be noted that the present invention is not limited to any particular material of the seal. For example, the second through hole may be sealed off with silicone. Alternatively, the sealing member may be implemented as a sealing ring.

For more details of the connection assembly 300 in the medical device of this embodiment, reference is made to the following description in connection with the second or third embodiment, and further description thereof is omitted here.

With continued reference to Fig. 2, in some exemplary implementations, the medical device may further include a sealing ring 510 disposed on the main enclosure 100 at a location where the upper shell 110 comes into abutment with the lower shell 120. In the medical device of this embodiment, the presence of the sealing ring 510 not only further improves the sealing performance of the inventive medical device and therefore additionally widens the range of activity places suitable for the wearer, but also dispenses with the need for any additional protective means, such as a disposable waterproof protective sheet. Further, this lays a foundation for allowing the wearer to take a bath at an appropriate time in the day.

With continued reference to Fig. 2, optionally, the medical device may further include a sealing pad 520 for sealing the first through hole that an electrode lead 600 is passed through. For example, optionally, the sealing pad 520 may include a silicone pad (not labeled) with a cross-shaped cut, and a connector of an electrode lead 600 may be passed through the cross-shaped cut and then electrically connected to the connection assembly 300. Through providing such a silicone pad with a cross-shaped cut, the sealing performance of the inventive medical device can be further enhanced, additionally broadening the range of activity places suitable for the wearer (e.g., humid environments like bathrooms).

With continued reference to Fig. 1, in some exemplary implementations, the patch assembly may include a single first patch 410 for fixing the main enclosure 100 at the target site. The first patch 410 may be detachably attached to the main enclosure 100. The detachable attachment of the first patch 410 to the main enclosure 100 allows easier replacement of the first patch 410 when needed.

Particularly, the attachment of the first patch 410 to the main enclosure 100 may be accomplished by at least one of a snap-fit, a snap fastener, adhesive bonding and a hook-and-loop fastener.

As shown in Fig. 1 or 2, the first patch 410 defines a fourth through hole (not labeled) for passage of an electrode lead 600 therethrough. The presence of the fourth through hole allows a connector of an electrode lead 600 to be passed into the interior of the main enclosure 100 through the fourth through hole and the sealing pad 520 (for sealing the first through hole) and then electrically connected to the connection assembly 300.

Optionally, in some exemplary implementations, the first patch 410 may be made from a 3M liquid dressing, a hydrogel dressing, a silicone dressing, an alginate dressing, a calcium alginate dressing or a hydrophilic fiber dressing, as well as an anti-inflammatory or antibiotic medicament. Specifically, considering the first patch 410 is used essentially to attach the main body of the medical device (e.g., a patch-type temporary pacemaker (P-TPG)) to the wearer's body surface, making it from a 3M liquid dressing, a hydrogel dressing, a silicone dressing, an alginate dressing, a calcium alginate dressing or a hydrophilic fiber dressing can impart to it air-permeable, waterproof, antibacterial and other desirable properties, which make it suitable for long-term attachment and wearing, for example, for longer than 3, 7, 15, 30 or another number of days. The anti-inflammatory or antibiotic medicament in the first patch 410 enables improved protection of a puncture site and reduces its risk of being infected. For example, the adhesion of the first patch 410 to the wearer's skin may degrade due to the wearer's physical activities and sweating, or for other reasons, leading to reduced reliability of the attachment or loss of adhesiveness. Alternatively, replacement of the first patch 410 may become necessary due to the wearer's allergic response or the like. In these cases, the first patch 410 may be torn off the main enclosure 100, and a new first patch 410 may be used to again fix the main enclosure 100 at the target site.

In other implementations, the patch assembly may include two first patches 410, which are separately detachably attached to the main enclosure 100 and used to fix the main enclosure 100 at the target site (e.g., on the wearer's body surface). A gap may be left between the two first patches 410, through which an electrode lead 600 can be passed. When multiple first patches 410 are used, some or all of them may be replaced, making the inventive medical device easier to use.

Of course, the patch assembly may include more first patches, such as three, four or even more first patches. In these cases, a gap for passage of an electrode lead 600 therethrough may be left between at least two of the patches. Further description in this regard is deemed unnecessary and omitted herein.

Reference is now made to Figs. 6 and 7. Fig. 6 shows an example layout of the medical device of this embodiment and an electrode lead 600 connected thereto inside the main enclosure 100. Fig. 7 shows another example layout of the medical device of this embodiment and an electrode lead 600 connected thereto inside the main enclosure 100. As can be seen from Figs. 6 and 7, there is an accommodation space for an electrode lead 600 within the interior of the main enclosure 100 of the inventive medical device. In some implementations, the accommodation space may be defined between the electrical protection enclosure 210 and the upper shell 110. Providing such an accommodation space for an electrode lead 600 makes the inventive medical device compatible with electrode leads 600 of various lengths.

Optionally, in some exemplary implementations, the medical device may further include cable auxiliary fixing members disposed in the main enclosure 100 (e.g., the stop tabs 211 as detailed below). With the cable auxiliary fixing members, an electrode lead 600 may be retained in a coiled state within the main enclosure 100. Otherwise, the coiled electrode lead 600 may uncoil and deform, possibly partly spreading out of the main enclosure 100. Thus, this arrangement facilitates operation of a physician.

With continued reference to Fig. 1 or 2, as an example, the cable auxiliary fixing members may include two opposing pairs of (i.e., totally four) stop tabs 211 arranged on the electrical protection enclosure 210 of the electrical assembly 200. Each of the stop tabs 211 may be curved toward the center of the electrical protection enclosure 210 so that an electrode lead 600 can be retained in a coiled state within the four stop tabs 211. The present invention is not limited to any particular material of the stop tabs 211. Optionally, they may be made of plastic or rubber.

In other specific implementations, a different number of stop tabs 211 (e.g., one, two or more) may be provided, and/or they may assume a different shape (e.g., straight) and/or be made of a different material. For example, a single stop tab 211 may be provided as a sheet-like structure having a U-shaped cross-section. This stop tab 211 can also provide the function of storing an electrode lead 600 in a coiled state. Further description in this regard is deemed unnecessary and omitted herein.

In other specific implementations, the cable auxiliary fixing members may also be provided as snap-fits, spring clips, cable ties, etc. From knowledge in the art, the skilled person will know how to configure and use snap-fits, spring clips and cable ties to retain an electrode lead 600, and further description thereof is therefore omitted herein.

In other specific implementations, the main enclosure 100 may consist of two separate sub-enclosures, in which the electrical assembly 200, the connection assembly 300 and a portion of an electrode lead 600 are housed. In these cases, the connection assembly 300 may be received in the same one of the sub-enclosures as the portion of the electrode lead 600. Designing the main enclosure 100 as two separate sub-enclosures allows each sub-enclosure to overall have a reduced height, improving the overall appearance of the wearer when he/she is wearing the medical device of this embodiment (compared with the one-piece unitary design of the main enclosure 100, each sub-enclosure is compacter in size and, when worn on the patient, easier to conceal due to a lower profile). In this way, it is made easier for the patient to perform physical activities. Further, the split-body design allows a physician to separately secure the individual modules of the medical device at more appropriate locations, depending on practical considerations.

Of course, the main enclosure 100 may also consist of more separate sub-enclosures, such as three, four or another number of sub-enclosures. This design can be flexibly adapted to various practical needs, and further description thereof is deemed unnecessary and omitted herein.

### EXAMPLE 2

In a second embodiment, there is provided a medical device, which is based on substantially the same principles as the medical device of the first embodiment. For the sake of brevity and conciseness, only differences of this second embodiment from the first embodiment and details of the connection assembly 300 that have not been set forth in the first embodiment will be described below. For more details of any previous-described feature that help appropriately understand the specific feature but are not mentioned in this embodiment, reference is made to the above description of the first embodiment in connection with the feature.

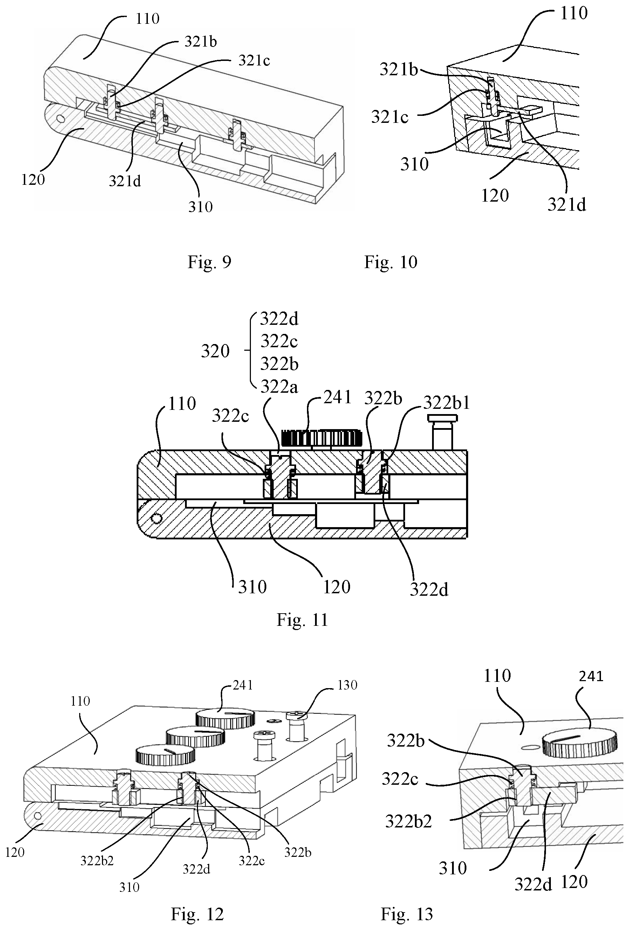

Particular reference is now made to Figs. 8, 9 and 10. Fig. 8 shows a cross-sectional view of an example of the connection assembly in the medical device of this embodiment, taken along a first direction. Fig. 9 shows a perspective view of an example of the connection assembly in the medical device of this embodiment partially cut away along the first direction. Fig. 10 shows a perspective partial view of an example of the connection assembly in the medical device of this embodiment partially cut away along a second direction (perpendicular to the first direction).

When comparing Figs. 8 to 10 that illustrate the medical device of this embodiment with Figs. 1 to 7 that illustrate the medical device of the first embodiment, it is easy to see that the main enclosure 100 in the medical device of this embodiment has a different shape from that of the main enclosure 100 in the medical device of the first embodiment - the main enclosure 100 in the medical device of this embodiment is rectangular, while the main enclosure 100 in the medical device of the first embodiment is hexagonal. Thus, the present invention is not limited to any particular shape of the main enclosure 100 in the inventive medical device. In alternative embodiments, the shape of the main enclosure 100 in the medical device may also be designed as an oblong enclosure, a flat circular enclosure, an irregularly shaped enclosure, among others, which will not be exhaustively listed here.

The connection assembly 300 is described in detail below. Continued reference is made to Fig. 8, 9 or 10. As can be seen from the figure, the connection assembly 300 includes at least one mounting recess 310 and at least one electrical connector 320. The mounting recess(es) 310 is/are configured to receive connector(s) of electrode lead(s) 600 therein so that the latter can be electrically connected to the electrical assembly 200 by the electrical connector(s) 320. Thus, in the inventive medical device, the mounting recess(es) 310 and the electrical connector(s) 320 can work together to clamp electrode connectors of different sizes, such as connectors of standard IS-1 electrode leads, connectors of floating electrodes for temporary pacing, connecting pins of temporary pacing electrodes, etc. In addition, the electrical connector(s) 320 can be kept in close contact with a connection cable and a contact in the connector of the electrode lead 600, ensuring the establishment of a reliable electrical connection. Further, after a connection is established with the control-system circuit module 230 using a connection cable, the current physiological state of the wearer's organ or tissue, can be monitored, and/or the physiological function (e.g., pacing and sensing) of the wearer's impaired organ or tissue can be enhanced or replaced.

It should be noted that the present invention is not limited to any particular correspondence between the mounting recess(es) 310 and the electrical connector(s) 320, and such correspondence may depend on the type(s) of electrode lead(s) 600 to be connected by the connection assembly 300.

In some implementations, each mounting recess 310 may correspond to a single electrical connector 320. In some other implementations, each mounting recess 310 may correspond to two or more electrical connectors 320. The following description is set forth in the context of each mounting recess 310 corresponding to two electrical connectors 320, as an example. In the case of each mounting recess 310 corresponding to another number of electrical connectors 320, a length of the mounting recess(es) 310 may increased or decreased to allow one or more than two electrical connectors 320 to be accommodated in each mounting recess 310. Further description in this regard is omitted herein, for the sake of brevity and conciseness.

In this embodiment, as shown in Fig. 8, in the connection assembly 300 of the medical device, the mounting recess(es) 310 is/are separate from the electrical connector(s) 320. For example, the mounting recess(es) 310 may be provided in the lower shell 120, and the electrical connector(s) 320 may be provided on the upper shell 110.

In particular, recessed accommodation portion(s) corresponding to the respective electrical connector(s) 320 may be provided in the upper shell 110 of the main enclosure 100, and the electrical connector(s) 320 may be received in the respective corresponding recessed accommodation portion(s). The mounting recess(es) 310 may be provided in the lower shell 120 of the main enclosure 100. Such a split-body design, in which the electrical connector(s) 320 of the connection assembly 300 is/are provided on the upper shell 110 and the mounting recess(es) 310 in the lower shell 120, not only facilitates the reception of a connector of an electrode lead 600 in a mounting recess 310, but also allows the connector of the electrode lead 600 to be preliminarily fastened in the mounting recess 310. Further, the electrical connector(s) 320 on the upper shell 110 can cooperate with the mounting recess(es) 310 in the lower shell 120 to more stably and more firmly maintain the main enclosure 100 in the closed configuration. Furthermore, since the electrical connector(s) 320 is/are partially received in the respective recessed accommodation portion(s), the main enclosure 100 is allowed to have a reduced dimension in the heightwise direction, laying a sound foundation for a compact shape and a light weight of the medical device.

Continued reference is made to Figs. 8, 9 and 10. As can be readily seen from Figs. 8, 9 and 10, in this embodiment, each recessed accommodation portion is provided by a blind hole 321a extending perpendicular to a plane, in which the upper shell 110 extends, and each electrical connector 320 includes a contact pin 321b, a connecting sheet 321d and a first spring 321c. The contact pin 321b defines an annular first spring engagement feature 321b1 extending around its circumference. The connecting sheet 321d is fixed to the upper shell 110 of the main enclosure 100 and extends in a direction forming a non-zero angle with the direction of extension of the contact pin 321b. In this embodiment, the angle between the directions of extension of the connecting sheet 321d and the contact pin 321b is 90°, and the contact pin 321b is inserted through the connecting sheet 321d. The first spring 321c is disposed over the contact pin 321b, with its opposite ends being respectively in abutment with the first spring engagement feature 321b1 and the blind hole 321a. In other specific implementations, the first spring 321c may be welded or otherwise processed at said location. Further description in this regard is deemed unnecessary and omitted herein.

For each electrical connector 320, when it is electrically connected to a connector of an electrode lead 600, the contact pin 321b is located at a first position, and the first spring 321c is in a first compressed configuration. In this configuration, the first spring 321c has a first length, and there is a gap between the first spring engagement feature 321b1 and the connecting sheet 321d, with an end of the contact pin 321b away from the blind hole 321a being in abutment with the connector.

For each electrical connector 320, when it is not connected to a connector of an electrode lead 600, the contact pin 321b is located at a second position, and the first spring 321c is in a second compressed configuration. In this configuration, the first spring 321c has a second length, and the first spring engagement feature 321b1 is in abutment with the connecting sheet 321d.

The second length is greater than the first length.

In the medical device of this embodiment, since part of the contact pin 321b and the first spring 321c in each electrical connector 320 is received in the respective blind hole 321a, the overall structure is made more compact. The contact pin 321b and the connecting sheet 321d in each electrical connector 320 can be used to electrically connect a connector of an electrode lead 600 to the electrical assembly 200 in the medical device (e.g., a P-TPG). Moreover, in each electrical connector 320, since the connecting sheet 321d oriented at a non-zero angle relative to the contact pin 321b is fixed to the upper shell 110, the contact pin 321b can be prevented from downward dislodgement from the blind hole 321a. Further, when a connector of an electrode lead 600 is inserted into any electrical connector 320, the first spring 321c can press the contact pin 321b against a contact in the connector, ensuring the establishment of a reliable electrical connection and making the electrical connector 320 suitably usable with electrode leads 600 of various sizes.

In other specific implementations, the first spring engagement feature 321b1 may be not necessarily annular, and may be alternatively in the form of an arc-shaped block. In the latter case, it can also desirably abut against the end of the first spring 321c. Additionally, the angle between the directions of extension of the connecting sheet 321d and the contact pin 321b is not limited to as described above. Alternatively, it may be 15°, 20°, 30° or another non-zero angle. However, preferably, the direction of extension of the contact pin 321b is parallel to the heightwise direction of the main enclosure 100, and the direction of extension of the connecting sheet 321d is parallel to the plane of extension of the main enclosure 100. Further description in this regard is deemed unnecessary and omitted herein.

It should be particularly noted that the contact pin 321b, the connecting sheet 321d and the first spring 321c in each electrical connector 320 in the connection assembly 300 may each be conductive or not, as described in the following two implementations.

In a first implementation, the contact pin 321b is conductive, while the first spring 321c and the connecting sheet 321d may be either conductive or not. In this case, the contact pin 321b is connected to a connection cable, and the contact pin 321b is pressed by the first spring 321c against, and thereby electrically connected to, a corresponding feature of a connector of an electrode lead 600.

In a second implementation, the contact pin 321b and the connecting sheet 321d are both conductive, while the first spring 321c may be either conductive or not. In this case, a connection cable is connected to the connecting sheet 321d, and a circumferential surface of the contact pin 321b is brought into an inner wall of a corresponding hole in the connecting sheet 321d, thereby electrically connecting the contact pin 321b to the connecting sheet 321d. In addition, the contact pin 321b is pressed by the first spring 321c against, and thereby electrically connected to, a corresponding feature of a connector of an electrode lead 600.

In both implementations, when the contact pin 321b is not connected to a connector of an electrode lead 600 (e.g., when the lower shell 120 and the upper shell 110 of the main enclosure 100 are in an opened configuration, or the electrode lead has not be inserted into the main enclosure yet), under the action of an upward force exerted by the connecting sheet 321d, the first spring 321c is in the second compressed configuration (in which the first spring 321c has the second length, and a lower end face of the first spring engagement feature 321b1 is in abutment with an upper end face of the connecting sheet 321d).

When the contact pin 321b is electrically connected to a connector of an electrode lead 600 (e.g., when the lower shell 120 and the upper shell 110 of the main enclosure 100 are closed, e.g., by a snap-fit, a screw, etc., with the electrode lead being inserted in the connection assembly), it is brought into close contact with a corresponding feature of the connector of the electrode lead 600 so as to provide a certain retention force. At the same time, the circumferential surface of the contact pin 321b is brought into close contact with, and thereby electrically connected to, the connecting sheet 321d, and the connecting sheet 321d is connected at the other end to a connection cable for the electrical assembly 200. In this way, it can be ensured that the connector of the electrode lead 600 is electrically connected to the electrical assembly 200.

Furthermore, in some exemplary implementations, the blind hole 321a may be optionally stepped, as particularly shown in Fig. 8, 9 or 10. As can be seen from Fig. 8, 9 or 10, the blind hole 321a may include a through section (not labeled) with a first radius and a blind section (not labeled) with a second radius. The two sections are coaxial with each other, and the first radius is greater than the second radius. Thus, a step is defined at an interface of the through section that has the first radius and the blind section that has the second radius. The opposite ends of the first spring 321c may abut respectively against the step and the first spring engagement feature 321b1. Through implementing the blind hole 321a as such a stepped one, on the one hand, one end of the first spring engagement feature 321b1 can be brought into abutment with the step, thus allowing the first spring 321c to have a reduced length and resulting in a cost reduction. Meanwhile, at least part of the first spring 321c can be received in the through section that has the first radius, ensuring that the first spring 321c, when compressed or tensioned, deforms along a straight line without deflections. On the other hand, the radially smaller section above the step (as viewed in the orientation of Fig. 8) can guide the contact pin 321b so as to prevent its tilting during reciprocation in the blind hole 321a, thereby extending the service life of the contact pin 321b. Of course, the radially larger section below the step (as viewed in the orientation of Fig. 8) may match a circumferential size of the first spring engagement feature 321b1, thereby also providing guidance to the contact pin 321b. Further description in this regard is deemed unnecessary and omitted herein.

### EXAMPLE 3

In a third embodiment, there is provided a medical device, which is based on substantially the same principles as the medical device of the first embodiment. For the sake of brevity and conciseness, only differences of this third embodiment from the first embodiment will be described below. For more details of any previous-described feature that help appropriately understand the specific feature but are not mentioned in this embodiment, reference is made to the above description of the first embodiment in connection with the feature.

Particular reference is now made to Figs. 11, 12 and 13. Fig. 11 shows a cross-sectional view of the medical device of this embodiment, taken along a third direction. Fig. 12 shows a perspective view of the medical device of this embodiment partially cut away along the third direction. Fig. 13 shows a perspective partial view of the medical device of this embodiment partially cut away along a fourth direction (perpendicular to the third direction).

When comparing Figs. 11 to 13 that illustrate the medical device of this embodiment with Figs. 1 to 7 that illustrate the medical device of the first embodiment, it is easy to see that the main enclosure 100 in the medical device of this embodiment has a different shape from that of the main enclosure 100 in the medical device of the first embodiment - the main enclosure 100 in the medical device of this embodiment is rectangular, while the main enclosure 100 in the medical device of the first embodiment is hexagonal. Thus, the present invention is not limited to any particular shape of the main enclosure 100 in the inventive medical device. In alternative embodiments, the shape of the main enclosure 100 in the medical device may also be designed as an oblong enclosure, a flat circular enclosure, an irregularly shaped enclosure, among others, which will not be exhaustively listed here.

When comparing Figs. 11 to 13 that illustrate the medical device of this embodiment with Figs. 1 to 7 that illustrate the medical device of the first embodiment, it is easy to also see that the locking mechanism 130 in the medical device of this embodiment differs in structure from the snap-fit type locking mechanism 130 in the medical device of the first embodiment. In this embodiment, the locking mechanism 130 in the medical device is a threaded structure, which can engage in threaded holes in the upper and lower shells, thereby connecting the two shell together. Thus, the inventive medical device is not limited to any particular structure of the locking mechanism 130. In further implementations, the locking mechanism 130 in the medical device may also be implemented as other structures, detailed description of which is, however, omitted herein.

The connection assembly 300 is described in detail below. Continued reference is made to Figs. 11, 12 and 13. As can be readily seen from the figures, in the illustrated exemplary implementations, each recessed accommodation portion is a through-hole structure 322a extending perpendicular to a plane in which the upper shell 110 extends. Each electrical connector 320 includes a set screw 322b, a conductive connecting block 322d and a second spring 322c. The set screw 322b defines an annular second spring engagement feature 322b1 extending around its circumference. The conductive connecting block 322d is fixed to the upper shell 110 of the main enclosure 100. The conductive connecting block 322d defines a third through hole in alignment with the through-hole structure. The set screw 322b has an external thread 322b2 in threaded engagement with the third through hole, and the second spring engagement feature 322b1 is located on a side of the conductive connecting block 322d away from the lower shell 120 of the main enclosure 100. The second spring 322c is disposed over the set screw 322b, with its opposite ends being respectively in abutment with the second spring engagement feature 322b1 and the conductive connecting block 322d. The set screw 322b can be rotated to adjust the position of the set screw 322b relative to the conductive connecting block 322d, thereby switching the electrical connector 320 between a connected state, in which it is electrically connected to a connector of an electrode lead 600, and a disconnected state.

In the medical device of this embodiment, through providing each recessed accommodation portion as a through-hole structure 322a extending perpendicular to the plane of extension of the upper shell 110, the position of the set screw 322b relative to the conductive connecting block 322d can be more easily adjusted using a supporting tool, making the electrical connector 320 suitably usable with connectors of various electrode leads 600. Moreover, since the set screw 322b is threadedly engaged with the conductive connecting block 322d, and because the conductive connecting block 322d is fixed to the upper shell 110 of the main enclosure 100, the set screw 322b is prevented from being ejected out of the upper shell 110 under the action of the second spring 322c and from dislodging from the through-hole structure 322a. Further, the set screw 322b and the conductive connecting sheet 321d can be operated to connect an electrical connection component of a connector of an electrode lead 600 to the electrical assembly 200 in the medical device (e.g., a P-TPG).

In other specific implementations, the second spring engagement feature 322b1 may also be of other shapes. For example, it may be in the form of an arc-shaped block, which is also suitable to abut against the end of the second spring 322c. Further description in this regard is deemed unnecessary and omitted herein.

Specifically, in order to disconnect the electrical connector 320 from a connector of an electrode lead 600 (e.g., by opening the lower shell 120 and the upper shell 110 of the main enclosure 100), the set screw 322b may be turned so as to move away from the mounting recess 310 relative to the conductive connecting block 322d, separating its lower end (as viewed in the orientation of Fig. 11) from the connector of the electrode lead 600. At this point, the second spring 322c may be in a first configuration, in which its lower end (as viewed in the orientation of Fig. 11) is in abutment with an upper end of the conductive connecting block 322d.

When the contact pin 321b is electrically connected to a connector of an electrode lead 600 (e.g., when the lower shell 120 and the upper shell 110 of the main enclosure 100 are closed, e.g., by a snap-fit, a screw, etc.), the second spring 322c is in a second configuration, in which it is compressed. Tightening the set screw 322b may cause it to move toward the mounting recess 310. When the set screw 322b is brought into close contact with a corresponding feature of the connector of the electrode lead 600 so as to provide a certain retention force, the set screw 322b will engage one end of the thread on the conductive connecting block 322d and therefore be electrically connected to the conductive connecting block 322d. The other end of the conductive connecting block 322d is connected to a connection cable for the electrical assembly 200. Thus, the connector of the electrode lead 600 is finally electrically connected to the electrical assembly 200.

Further, in some optional implementations, a section of the through-hole structure 322a above the set screw 322b (as viewed in the orientation of Fig. 11) may be provided therein with a sealing and insulating feature, such as a silicone pad with a cut. In these cases, a tightening tool such as a torque wrench may be pressed down through the cut in the silicone pad to access the set screw 322b. After the set screw 322b is turned as desired, the tightening tool may be withdrawn from the through-hole structure 322a, and the cut in the silicone pad may again close, providing sealing and insulation at the upper end of the through-hole structure 322a. The sealing silicone pad may be made from a medical dressing with waterproof and insulating properties. Further description thereof is deemed unnecessary and omitted herein.

Further, in some optional implementations, the through-hole structure 322a may be optionally stepped, as particularly shown in Fig. 11, 12 or 13. The through-hole structure 322a may include a through section (not labeled) with a third radius and another through section (not labeled) with a fourth radius. The two sections are coaxial with each other. The through section that has the third radius is closer to the conductive connecting block 322d, and the third radius is greater than the fourth radius. Thus, a step is defined at an interface of the through section that has the third radius and the through section that has the fourth radius. When the set screw 322b is rotated so as to move away from the mounting recess 310, the second spring engagement feature 322b1 will come into abutment with the step of the through-hole structure 322a, preventing the set screw 322b from further movement along an axis of the through-hole structure 322a out of the conductive connecting block 322d. That is, the set screw 322b is prevented from moving beyond a limit defined by the conductive connecting block 322d during its rotation.

It should be particularly noted that the present invention is not limited to any particular layout of the electrical assembly 200 and the connection assembly 300 inside the main enclosure 100. In the case of the main enclosure 100 being of a flip-top type, as an example, in some implementations, the electrical assembly 200 may be disposed closer to the articulated joint of the upper shell 110 and the lower shell 120, while the connection assembly 300 may be arranged farther away from the articulated joint of the upper shell 110 and the lower shell 120. In some other implementations, the electrical assembly 200 may be disposed farther away from the articulated joint of the upper shell 110 and the lower shell 120, while the connection assembly 300 may be arranged closer to the articulated joint of the upper shell 110 and the lower shell 120. Other layouts are also possible, and further description thereof is deemed unnecessary and omitted herein.

### EXAMPLE 4

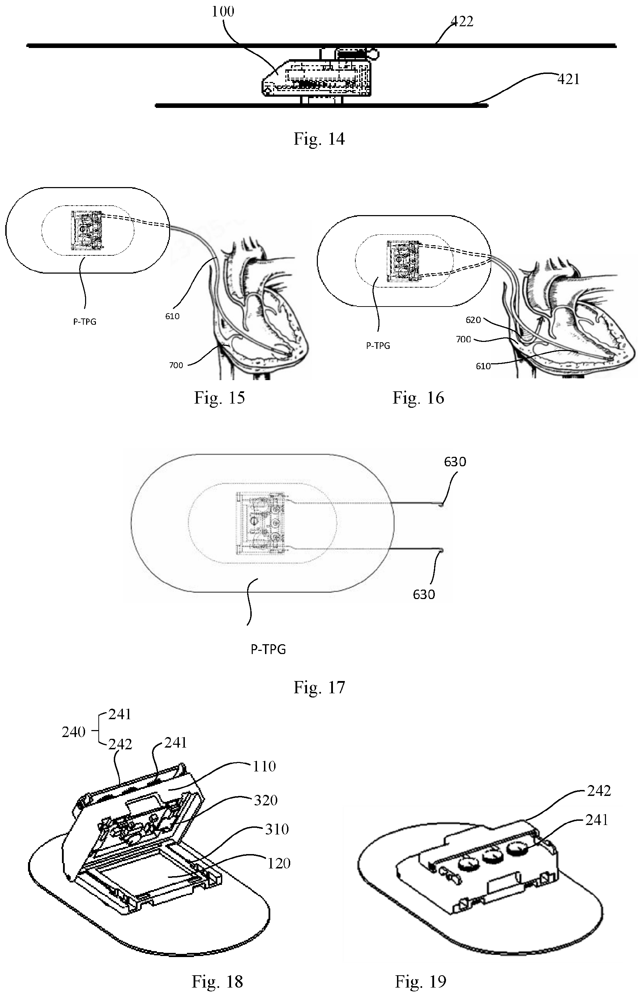

In a fourth embodiment, there is provided a medical device, which is based on substantially the same principles as the medical devices of the second to third embodiments. For the sake of brevity and conciseness, only differences of this fourth embodiment from the second and third embodiments will be described below. For more details of any previous-described feature that help appropriately understand the specific feature but are not mentioned in this embodiment, reference is made to the above description of the second to third embodiments in connection with the feature. Particular reference is now made to Fig. 14, which shows an elevational view of an example of the medical device of this embodiment. As can be readily seen from Fig. 14, the medical device of this embodiment differs from those of the second to third embodiments in that the patch assembly in the medical device of this embodiment includes a main patch 421 attached to a wearer's body surface and an auxiliary adhesive patch 422.

In particular, the main enclosure 100 in the medical device and a portion of an electrode lead 600 may be sandwiched between the main patch 421 and the auxiliary adhesive patch 422. With this arrangement, the main patch 421 can fixedly attach a main body of the medical device (the other portion thereof than the patch assembly) at a target site (e.g., on the wearer's body surface), and an electrode lead 600 may be secured between the main patch 421 and the auxiliary adhesive patch 422. Optionally, a projection of the main patch 421 on the main enclosure 100 is encompassed by a projection of the auxiliary adhesive patch 422 on the main enclosure 100. With this arrangement, peripheral portions of the auxiliary adhesive patch 422 (which extend beyond the main patch 421) will come into contact with the wearer's body surface, helping in attaching the main body of the medical device.

Optionally, materials suitable for making the main patch 421 may include air-permeable materials, and materials suitable for making the auxiliary adhesive patch 422 may include waterproof materials. When the main patch 421 is made of an air-permeable material, it can improve wearing comfort of the inventive medical device. When the auxiliary adhesive patch 422 has waterproof properties, protection against water can be provided for the main body of the medical device.

For example, when the medical device of this embodiment is implemented as a temporary pacemaker (e.g., a P-TPG), suitable connectors of electrode leads 600 may include, but are not limited to, connectors of standard IS-1 electrode leads, connectors of floating electrodes for temporary pacing, and connecting pins of temporary pacing electrodes. Particular reference is now made to Figs. 15 to 17. Fig. 15 schematically illustrates an example of the medical device of this embodiment being connected to an implantable IS-1 ventricular electrode lead. As can be seen from Fig. 15, in this example, when implemented as a patch-type temporary pacemaker (P-TPG), the inventive medical device may be connected to an IS-1 ventricular electrode lead 610 and used for temporary single-chamber (ventricular) pacing of the wearer's heart 700. Fig. 16 schematically illustrates another example of the medical device of this embodiment being connected to implantable IS-1 ventricular and atrial electrode leads. As can be seen from Fig. 16, in this example, when implemented as a patch-type temporary pacemaker (P-TPG), the inventive medical device may be connected to an IS-1 ventricular electrode lead 610 and an IS-1 atrial electrode lead 620 and used for temporary dual-chamber (atrial and ventricular) pacing of the wearer's heart 700. Fig. 17 schematically illustrates yet another example of the medical device of this embodiment being connected to a conventional temporary pacing electrode lead. As can be seen from Fig. 17, in this example, when implemented as a patch-type temporary pacemaker (P-TPG), the inventive medical device may be connected to a temporary electrode lead 630 and used for temporary pacing of the wearer's heart 700.

### EXAMPLE 5

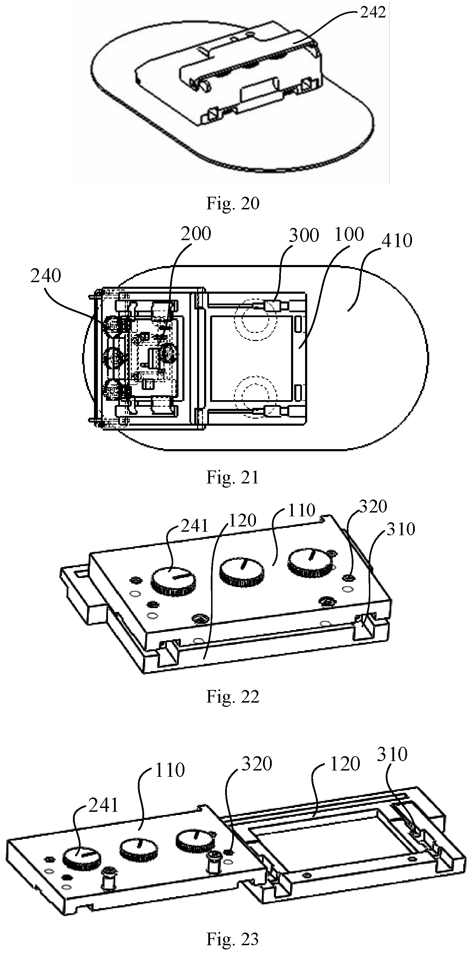

In a fifth embodiment, there is provided a medical device, which is based on substantially the same principles as the medical devices of the first to third embodiments. For the sake of brevity and conciseness, only differences of this fifth embodiment from the first to third embodiments will be described below. For more details of any previous-described feature that help appropriately understand the specific feature but are not mentioned in this embodiment, reference is made to the above description of the first to third embodiments in connection with the feature. The differences from the first to third embodiments can be particularly understood from Figs. 18 to 20. Fig. 18 schematically illustrates opened upper and lower shells in an example of the third embodiment of the present invention. Fig. 19 schematically illustrates the main enclosure of the medical device of Fig. 18 in a closed configuration and a protective feature for preventing accidental touching in an opened configuration. Fig. 20 schematically illustrates the main enclosure of the medical device of Fig. 18 in the closed configuration and the protective feature for preventing accidental touching also in a closed configuration.

The medical device of this embodiment further includes a tuning assembly 240 provided on an outer wall surface of the main enclosure 100. The tuning assembly 240 includes at least one adjustment knob 241 electrically connected to the electrical assembly 200. An operator can manually tune a parameter (e.g., pacing rate) of the medical device (e.g., a patch-type pacemaker) as needed, by manipulating the adjustment knob 241. For example, the adjustment knob 241 may be optionally suitable for manual manipulation.

In order to prevent the adjustment knob 241 from being accidentally touched, the tuning assembly 240 may further include a protective feature 242 for preventing accidental touching, which can increase safety of the medical device during use. In this embodiment, the protective feature 242 may be particularly implemented as a separate inverted-U-shaped protective hood, which is disposed over the adjustment knob 241 and slidably attached to the main enclosure 100. Thus, the adjustment knob 241 is switchable between a hidden and protected state and an exposed state.

It should be noted that, other implementations are possible for the protective feature 242, and the present invention is not limited to any particular implementation.

### EXAMPLE 6

In a sixth embodiment, there is provided a medical device, which is based on substantially the same principles as the medical device of the third embodiment. For the sake of brevity and conciseness, only differences of this sixth embodiment from the third embodiment will be described below. For more details of any previous-described feature that help appropriately understand the specific feature but are not mentioned in this embodiment, reference is made to the above description of the third embodiment in connection with the feature. Particular reference is now made to Figs. 21, 22 and 23. Fig. 21 shows a schematic overall view of the medical device of this embodiment. Fig. 22 is a schematic illustration of a main enclosure 100 in the medical device of this embodiment, which is being in a closed configuration. Fig. 23 is a schematic illustration of the main enclosure 100 in the medical device of Fig. 22, which is being in an opened configuration. As can be readily seen from Figs. 21, 22 and 23, in the main enclosure 100 of the medical device of this embodiment, an upper shell 110 is slidably coupled to a lower shell 120, making the main enclosure 100 of a slide-top type. The main enclosure 100 further includes two locking mechanisms 130. In the present implementation, the locking mechanisms 130 are threaded structures. In alternative implementations, there may be a different number of locking mechanisms 130. When closed together, the upper shell 110 and the lower shell 120 can be locked to each other using the locking mechanisms 130. In the medical device of this embodiment, the slide-top design of the main enclosure 100 can effectively reduce a number of inconveniences associated with flipping open of the upper shell 110 and allows the upper and lower shells to be closed more securely. With the locking mechanisms 130, the main enclosure 100 can be more steadily maintained in the closed configuration. From knowledge in the art, the skilled person can obtain more details of such a slide-top enclosure, further description thereof is deemed unnecessary and omitted herein.

### EXAMPLE 7

In a seventh embodiment, there is provided a medical device control system, which includes a medical device according to any of the first to sixth embodiments discussed above and a medical programming device communicatively connected to the medical device. Since the medical device control system of this embodiment belongs to the same inventive concept as the medical devices of the foregoing embodiments, it has at least all the advantages of the medical devices of the foregoing embodiments. For brevity, these advantages are not repeated here and reference is made to the above description in connection with the medical devices for more details.

As an example, in the medical device control system of this embodiment, the following procedure may be followed to set parameters of the medical device (for ease of description, the medical device is described hereinafter in the context of being implemented as a P-TPG, as an example).
1) A P-TPG that can address a wearer's need is chosen, and its parameters are fixed, by medical personnel (e.g., a physician). After a connection is established, the P-TPG operates with the fixed parameters.
2) During operation of the P-TPG, a physician may modify any one or more of the parameters through wireless (e.g., Bluetooth) communication, if doing so is necessary for the wearer. After a connection is established, the P-TPG will operate with the updated parameter(s). This process may be repeated for any subsequent parameter change.
3) If parameters are to be adjusted mechanically, e.g., by manipulating a adjustment knob (e.g., a manual adjustment knob), a corresponding protective feature for preventing accidental touching (e.g., an inverted-U-shaped protective plate) may be opened before the knob can be manipulated. After a connection is established, a physician may adjust one or more of the parameters, as needed by the wearer. After a connection is established, the P-TPG will operate with the updated parameters. The protective feature (e.g., an inverted-U-shaped protective plate) is adapted to prevent the wearer or others from accidentally touching or otherwise undesirably interacting with the adjustment knob. Likewise, if any need arises subsequently for a parameter change, a physician may open the protective feature (e.g., an inverted-U-shaped protective plate) and reconfigure the parameter by manipulating the adjustment knob.

Reference throughout this specification to "one embodiment", "some embodiments", "an example", "a specific example" "some examples" or the like means that a particular feature, structure, material, or characteristic described in connection with the embodiments or examples is included in at least one embodiment or example of the present application. Thus, the appearances of those phrases in various places throughout this specification are not necessarily referring to the same embodiment or example of the application. Additionally, the particular features, structures, materials, or characteristics may be combined in any suitable manner in any one or more embodiments. Further, should there be no contradiction, one of ordinary skill in the art can combine the various embodiments, examples and features thereof described herein in any combination.

The present invention offers the following benefits over the prior art:
It provides a medical device including a main enclosure, an electrical assembly, a connection assembly and a patch assembly. The electrical assembly and the connection assembly are arranged in the main enclosure, and the main enclosure defines a first through hole adapted for passage of an electrode lead therethrough. The connection assembly is configured to be electrically connected to the electrical assembly and the electrode lead. The patch assembly is configured to fix at least one of the main enclosure and the electrode lead at a target site (for example, when the patch assembly is used to fix the main enclosure, the target site may be on the body surface of a wearer of the medical device). Thus, through integrating the main enclosure, the electrical assembly, the connection assembly and the patch assembly, the medical device of the present invention combines the advantages of both implantable medical devices (e.g., implantable pacemakers) and temporary medical devices (e.g., conventional temporary pacemakers) in the related art. With this design, the inventive medical device is lighter in weight, compacter in size, more easily wearable by a wearer and associated with a lower risk of infection, and is therefore more favorable to the wearer's recovery. Moreover, through housing the electrical assembly and the connection assembly in the main enclosure, not only protection can be provided for the electrical assembly and the connection assembly, but a main body of the medical device (consisting of the main enclosure, the electrical assembly and the connection assembly) can be worn on the wearer through the patch assembly. Additionally, since the main body of the medical device is lightweight and compact, it can be worn, for example, via a back or other strap, or even placed within a pocket. Thus, the patch assembly can be used to fix an electrode lead and take care of and provide protection for an incision made at a puncture site (i.e., the target site), reducing the risk of infection at the puncture site. Further, in the medical device of the invention, the connection assembly can be suitably used with a variety of electrode leads (e.g., it is adapted to mate with connectors of standard IS-1 electrode leads, connectors of floating electrodes for temporary pacing, connecting pins of temporary pacing electrodes, etc.) Because of such applicability to more electrode leads, a physician is allowed to more easily make an appropriate recovery plan (e.g., preventive, therapeutic or temporary (provisional), using the proposed inventive medical device) that matches the actual need of an individual wearer.

The present invention also provides a medical device control system, which belongs to the same inventive concept as the medical device of the invention and thereby has at least all the advantages of the inventive medical device. For brevity, these advantages are not repeated here, and reference is made to the above description in connection with the medical device for more details.

Various medical device and medical device control system configurations disclosed herein have been described in detail above with respect to the foregoing embodiments. Of course, the above description is merely that of a few preferred modes of carrying out the invention and is in no way intended to limit the scope thereof. Possible configurations of the present invention include, but are not limited to, those described in the foregoing embodiments, and those skilled in the art can obtain in light of the above teachings. Accordingly, any and all variations and modification made by those of ordinary skill in the art to which the present invention pertains in light of the above teachings are intended to fall within the scope thereof as defined by the appended claims.

## Claims

1. A medical device, comprising a main enclosure, an electrical assembly, a connection assembly and a patch assembly, both the electrical assembly and the connection assembly arranged in the main enclosure, the main enclosure provided with a first through hole for an electrode lead to pass through,
the connection assembly configured to be electrically connected to the electrical assembly and the electrode lead, and
the patch assembly configured to fix at least one of the main enclosure and the electrode lead to a target site.

2. The medical device according to claim 1, wherein the electrical assembly comprises an electrical protection enclosure, a power supply and a control-system circuit module electrically connected to the power supply, wherein the power supply and the control-system circuit module are disposed in a closed accommodation space delimited by the electrical protection enclosure and the main enclosure, and
wherein the power supply is configured to provide electrical power to the control-system circuit module.

3. The medical device according to claim 2, wherein the electrical assembly further comprises a connection cable, wherein the control-system circuit module is electrically connected to the connection assembly through the connection cable, and wherein the electrical protection enclosure defines a second through hole therein adapted for passage of the connection cable therethrough.

4. The medical device according to claim 3, wherein the electrical protection enclosure further comprises a sealing member for sealing a gap between the second through hole and the connection cable.

5. The medical device according to claim 1, wherein the connection assembly comprises at least one mounting recess and at least one electrical connector, wherein the at least one mounting recess is configured to receive a connector of the electrode lead therein, and wherein the connector of the electrode lead is electrically connected to the electrical assembly by the at least one electrical connector.

6. The medical device according to any one of claims 1 to 5, wherein the main enclosure comprises an upper shell, a lower shell and a locking mechanism, wherein the upper shell is articulated to the lower shell, making the main enclosure of a flip-top type, or wherein the upper shell is slidably connected to the lower shell, making the main enclosure of a slide-top type; and
wherein when the upper shell and the lower shell are closed, the upper shell and the lower shell are fixedly connected by the locking mechanism.

7. The medical device according to claim 5, wherein the upper shell of the main enclosure is provided with at least one recessed accommodation portion corresponding to the respective electrical connector one-to-one, wherein the at least one electrical connector is partially received in respective recessed accommodation portion, and wherein the at least one mounting recess is provided in the lower shell of the main enclosure.

8. The medical device according to claim 7, wherein each of the at least one recessed accommodation portion is a blind hole extending perpendicular to a plane in which the upper shell extends,
wherein each electrical connector comprises:
a contact pin, an outer periphery of the contact pin provided with a first spring engagement feature;
a connecting sheet fixed on the upper shell of the main enclosure, which extends in a direction forming a non-zero angle with a direction of extension of the contact pin, and wherein the contact pin passes through the connection sheet; and
a first spring, which is disposed over the contact pin, opposite ends of the first spring are respectively in abutment with the first spring engagement feature and the blind hole,
wherein when the electrical connector is electrically connected to the connector of the electrode lead, the contact pin is located at a first position, and the first spring is in a first compressed configuration, in which the first spring has a first length, the first spring engagement feature has a gap with the connecting sheet, and an end of the contact pin away from the blind hole abuts against the connector;
when the electrical connector is disconnected from the connector of the electrode lead, the contact pin is located at a second position, and the first spring is in a second compressed configuration, in which the first spring has a second length, and the first spring engagement feature abuts against the connecting sheet; and
wherein the second length is greater than the first length.

9. The medical device according to claim 7, wherein each of the at least one recessed accommodation portion is a through-hole structure extending perpendicular to a plane in which the upper shell extends,
wherein each of the at least one electrical connector comprises:
a set screw, an outer periphery of the set screw is provided with a second spring engagement feature;
a conductive connecting block fixed on the upper shell of the main enclosure, the conductive connecting block is provided with a third through hole at a position in alignment with the through-hole structure, the set screw is threadedly connected to the third through hole, and the second spring engagement feature is located on a side of the conductive connection block away from the lower shell of the main enclosure; and
a second spring, which is disposed over the set screw, opposite ends of the second spring are respectively connected to the second spring engagement feature and the conductive connecting block, and
wherein by rotating the set screw to change a position of the set screw relative to the conductive connecting block, the electrical connector is switched between a connected state, in which the electrical connector is electrically connected to the connector of the electrode lead, and a disconnected state.

10. The medical device according to claim 1, further comprising a cable auxiliary fixing member arranged in the main enclosure.

11. The medical device according to claim 1, further comprising:
a sealing ring arranged at a location where the upper shell and the lower shell of the main enclosure come into abutment with each other; and/or
a sealing pad for sealing a gap between the electrode lead and the first through hole, the sealing pad comprising a silicone pad having a cross-shaped cut therein, wherein the connector of the electrode lead passes through the cross-shaped cut and then is connected to the connection assembly.

12. The medical device according to claim 1, wherein the patch assembly comprises at least one first patch, which is adapted to fix the main enclosure at the target site, and wherein the first patch is detachably connected to the main enclosure.

13. The medical device according to claim 12, wherein the patch assembly comprises a single tearable first patch, the first patch has a fourth through hole therein, which is adapted for passage of the electrode lead therethrough, or
wherein the patch assembly comprises at least two first patches, which are individually detachably connected to the main enclosure and thereby fix the main enclosure at the target site, and wherein a gap allowing passage of the electrode lead therethrough is left between the at least two first patches.

14. The medical device according to claim 1, wherein the patch assembly comprises a main patch and an auxiliary adhesive patch capable of connecting to the target site, and wherein the main enclosure and a portion of the electrode lead are disposed between the main patch and the auxiliary adhesive patch.

15. The medical device according to claim 1, wherein the main enclosure is separate from the patch assembly, wherein the main enclosure is wearable, and wherein the patch assembly comprises a second patch for fixing the electrode lead to the target site.

16. The medical device according to claim 1, wherein the main enclosure comprises at least two separate sub-enclosures, wherein the electrical assembly, the connection assembly and a portion of the electrode lead are accommodated in the at least two separate sub-enclosures, and wherein the connection assembly and the portion of the electrode lead are located in a single one of the sub-enclosures.

17. The medical device according to claim 1, further comprising a tuning assembly disposed on an outer wall of the main enclosure, and wherein the tuning assembly comprise at least one adjustment knob electrically connected to the electrical assembly.

18. A medical device control system, comprising a medical programming device and the medical device according to any one of claims 1 to 17, which is communicatively connected to the medical programming device.
